(19) Europäisches Patentamt
European Patent Office
Office européen des brevets

(11) **EP 2 177 272 B1**

(12) **EUROPEAN PATENT SPECIFICATION**

(45) Date of publication and mention
of the grant of the patent:
**28.10.2015  Bulletin 2015/44**

(51) Int Cl.:
***B05B 1/02*** *(2006.01)*

(21) Application number: **09178981.8**

(22) Date of filing: **28.08.2001**

(54) **Nozzle device and nozzle for atomisation and/or filtration and methods for using the same**

Filtrier- und/oder Emulgiervorrichtung sowie Verfahren zu ihrer Verwendung

Dispositif de filtrage et/ou émulsification, ainsi que son procédé d'utilisation

(84) Designated Contracting States:
**AT BE CH CY DE DK ES FI FR GB GR IE IT LI LU MC NL PT SE TR**

(30) Priority: **28.08.2000  NL 1016030**

(43) Date of publication of application:
**21.04.2010  Bulletin 2010/16**

(62) Document number(s) of the earlier application(s) in accordance with Art. 76 EPC:
**01974998.5 / 1 315 573**

(73) Proprietor: **Aquamarijn Holding BV**
**7255 DB  Hengelo (NL)**

(72) Inventors:
• **Van Rijn, Cornelis Johannes Maria**
**7255 DB, Hengelo (NL)**
• **Nijdam, Wietze**
**7201 JE, Zutphen (NL)**

(74) Representative: **Jilderda, Anne Ayolt**
**Octrooibureau LIOC B.V.**
**Postbus 13363**
**3507 LJ  Utrecht (NL)**

(56) References cited:
**US-A- 6 086 195**

**Description**

*The present invention relates to a device for filtration and/or emulsification of a fluid, comprising a nozzle plate and a nozzle plate support body, said nozzle plate support body carrying said nozzle plate at a main surface side, wherein said nozzle plate has a thickness of less than 2 micron and comprises a plurality of nozzle orifices extending over said thickness through said nozzle plate, wherein said support body comprises a cavity and said plurality of nozzle orifices are jointly in fluid communication with said cavity, and wherein a nozzle orifice within said plurality of nozzle orifices has a length which is less than six times its diameter, and in particular is shorter than its diameter.* The invention further relates to methods applying such a filtration and/or emulsification device.

These devices are used for filtration purposes and or for atomisation of a fluid to produce small liquid droplets in air (spray) or into a liquid (emulsion) with a relatively narrow droplet size distribution and to make small air bubbles into a liquid (foam) and to methods of using the same. The device and especially the nozzle plate may be produced by micro-machining (Micro System Technology) which means that the subject nozzle part means are produced using lithography steps related to semiconductor fabrication methods. Alternatively spark erosion and laser drilling techniques may be used, but in general these tend to be less reproducible and less precise in comparison with micro-machining methods.

US patent 6.086.195 *discloses a device as described in the opening paragraph for filtration purposes in an inkjet printing head. This known device comprises a filter carrier which supports a micro-screen filter. The filter carrier is adapted to provide stability, support and reinforcement to the micro-screen filter. As such, the known filter carrier is preferably made of a material, such as stainless steel, to provide the suitable support and reinforcement to the micro-screen filter and also is securely coupled to the micro-screen filter. Cavities are provided in the filter carrier to expose the micro-screen filter and to offer fluid communication with the orifices which are provided in said micro-screen filter. These orifices are evenly distributed over the micro-screen filter an extend all the way from each edge of said cavities to each opposite edge.*

*To this end a nozzle device as described in the opening paragraph is according to the invention is characterized in that said nozzle orifices of said plurality of nozzle orifices are arranged in a first zone of said nozzle plate, in that said nozzle plate comprises a second zone adjacent said first zone which is at least substantially free of any nozzle orifice, in that said cavity extends underneath both said first zone and said second zone of said nozzle plate, and in that said second zone has a width which is at least a number of times as large as said thickness of said nozzle plate.*

Yet another object of the invention is to provide atomising devices that are less sensitive for the Pascal threshold pressure.

These and additional objects and advantages of the invention will become apparent from the technical description which follows.

It is to be understood that both the foregoing summary and the following technical description are exemplary and explanatory and are not restrictive of the invention as claimed.

BRIEF DESCRIPTION OF THE DRAWINGS

Fig.1      is a cross section of a nozzle device with a nozzle plate and pre-filter for atomisation.
Fig.2      is a cross section of a nozzle device with a nozzle plate and pre-filter for atomisation, in which the nozzle plate and the pre filter are deepened for protection.
Fig.3      is a cross section of a nozzle device with a nozzle plate and pre-filter for atomisation made from one piece.
Fig.4      is a top view of a nozzle plate containing slits plus special slits for pressure reduction.
Fig.5A      is a cross section of a nozzle device containing more orifices to increase the throughput.
Fig.5B      is a cross section of a nozzle device containing more orifices to increase the throughput with the possibility of liquid flow on both sides of the membrane.
Fig.6      is a top view of closely packed nozzle plates.
Fig.7A      is a top view of interconnected nozzle plates.
Fig.7B      is a cross section of interconnected nozzle plates.
Fig.8A      is a cross section of a thick nozzle plate with reduced flow resistance.
Fig.8B      is a cross section of a spiral nozzle orifice.
Fig.8C      is a cross section of the manufacturing method of a spiral nozzle orifice.
Fig.9      is a top view of nozzle plate with improved jetting behaviour.
Fig.10A      is a top view of a substantially stronger nozzle plate with slit type orifices.
Fig.10B      is a top view of a substantially stronger nozzle plate with circular orifices.
Fig.11      is a cross section of a nozzle device with a coated nozzle plate.

Fig.12    is a top view of a nozzle plate with separated hydrophilic/hydrophobic membrane coating for improved jetting/jet start.

Fig.13    is a cross section of a nozzle plate with slightly protruding nozzles.

Fig.14    is a cross section of a nozzle plate with hydrophilic/hydrophobic coatings around the orifices.

Fig.15    is a cross section of a vibrating nozzle plate with drain plate.

Fig.16    is a cross section of nozzle plate, placed under an angle, in a cross flow channel.

Fig.17    is a top view of a nozzle plate with extra nozzles for co-flow.

Fig.18    is a cross section of a nozzle plate for emulsification.

Fig.19    is a cross section high performance filter with the possibility for light to pass through.

Fig.20    is a top view of a filter for analysing particles that isolates the particles for enhanced recognition.

Fig.21    is a top view of a plastic disc supporting a nozzle plate for analysis purposes.

Fig.22    is a cross section of a plastic disc supporting a nozzle plate for analysis purposes plus funnel.

Fig.23    is a cross section of a reusable nozzle plate with a transparent cross flow channel.

Fig.24    is a top view of a glass module plate for high volume nozzle plates.

Fig.25    is a cross section of a glass module plate for high volume nozzle plates showing the shallow cross flow channels.

Fig.26    is a cross section of a glass module plate for high volume nozzle plates showing a channel of the comb structure.

Fig.27A    is a cross section of stacked module plates.

Fig.27B    is a opened 3D view of stacked module plates showing the liquid flow within the stack.

Fig.28    is cross section of a nozzle plate for evaporation purposes.

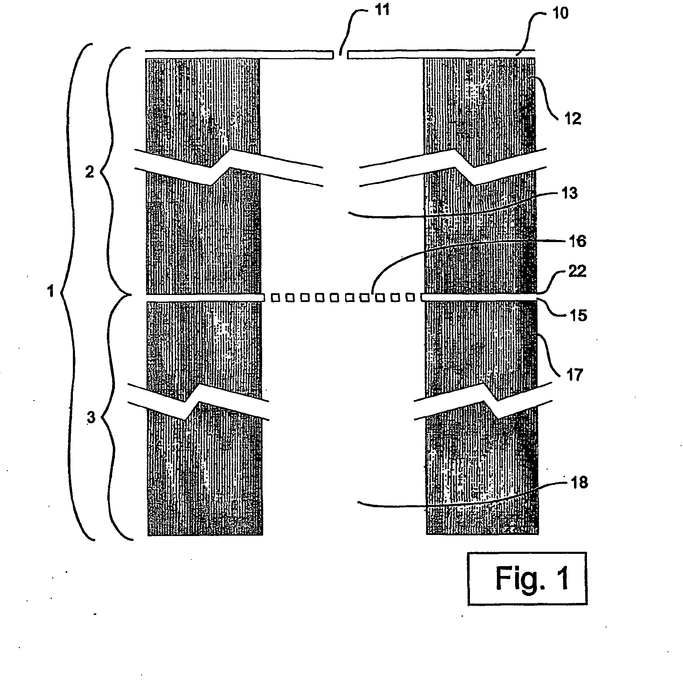

A first embodiment of a nozzle device 1 is shown in FIG. 1. The nozzle device 1 comprises a nozzle for atomisation 2, with a nozzle plate 10 with at least one nozzle orifice 11 and a nozzle plate support body 12 with a nozzle cavity 13, further comprising filtration means 3 with a filtration plate 15 with at least one filtration orifice 16 and a filtration plate support body 17 with at least one filtration cavity 18. This nozzle device 1 is rather insensitive for microbiological fouling and unwanted leakage due to blocked nozzle orifices 11 because of the placement of a pre-filter for the nozzle for atomisation 2.

Basically the nozzle for atomisation 1 and filtration means 2 are made with the same micro machining techniques giving many additional advantages. The two parts 2 and 3 may have similar size and flatness and can therefor easily be directly bonded or glued 22 together without the need of separate or elaborated connection parts, that may introduce particle contamination between the nozzle plate 11 for atomisation and filtration plate 15. A silicon wafer containing a number of nozzles for atomisation and a silicon wafer with a number of filtration means may be first bonded together before sawing the wafer sandwich into separate dies with individual nozzle and filtration means.

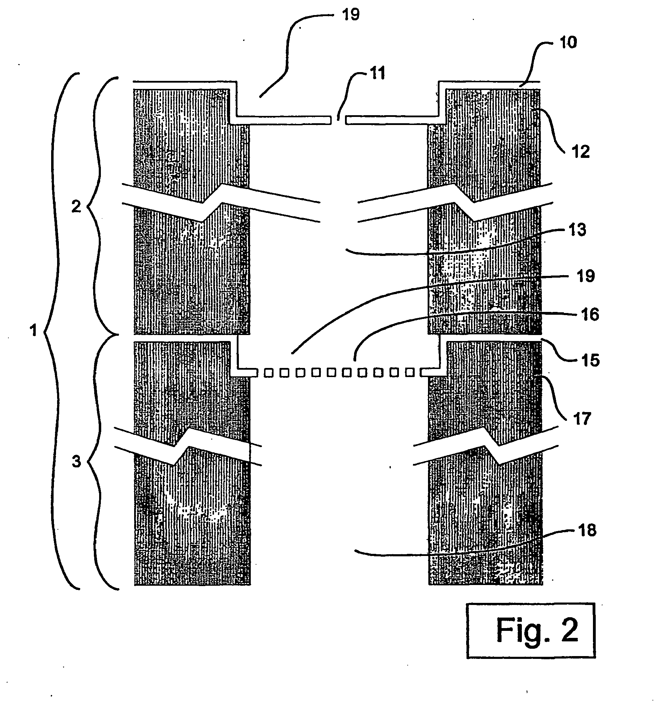

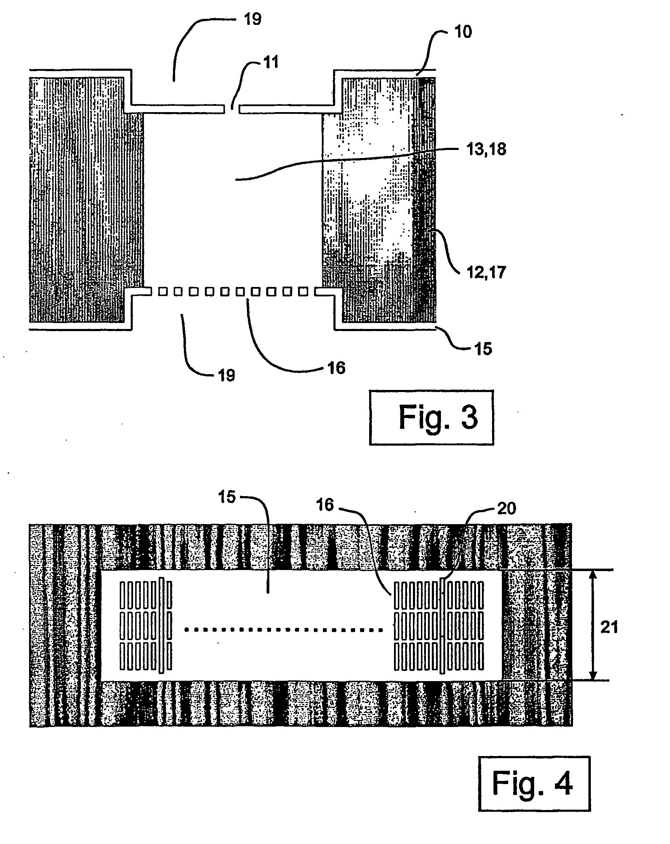

Another embodiment of a nozzle device 1 is shown in FIG. 2, which is **characterised in that** the nozzle orifices 11, 16 are made in a 2-200 micron deepened region 19 of the nozzle plate 2,3 with respect to the nozzle plate support body 12 and filtration plate support body 17, herewith protecting the nozzle and/or filtration means during manufacturing and assembly against scratches etc. With preference the nozzle plate support body 12 and filtration plate support body 17 are identical, FIG. 3, the cavities 13,18 are then made by etching the support material directly through the nozzle and filtration orifices 11,16. With this manufacturing process there can not be any particle contamination between the filtration plate 15 and the nozzle plate for atomisation 10.

In some cases it has been proven useful to make in the filtration means 3 one or more filtration orifices substantially larger (1-3 micron) than the other ones (0.2-0.8 micron) in order to reduce the Pascal pressure or to facilitate the removal of etching material(gas).

Alternatively the filtration orifices 16 are slit-shaped to reduce the Pascal pressure. A special embodiment of such a filtration plate has a number of very long slit shaped filtration orifices 20 (e.g. with a length of 50-100 micron) near the edges of the filtration plate. Depending on the width 21 of the filtration plate 15 (e.g. 100 micron) and the applied pressure (e.g. 1 bar) this slit will open due to local bending of the filtration plate 15 (FIG. 4). Preferably the fluid resistance of the filtration plate 15 is minimal 3 times smaller than the fluid resistance of the nozzle plate 10. By this the pressure across the nozzle device 1 is effectively only used for atomisation.

Nozzles for atomisation 2 can be made with known micro machining techniques. A mono crystalline silicon wafer 12 with thickness 400 micron is provided with a Low Pressure Chemical Vapour Deposition grown layer 10 of low stress silicon nitride with a thickness of 1 micron. With a suitable mask a photo lacquer pattern with 2 micron orifices at the front side of the wafer 12 and a similar pattern with 15 micron openings at the back side is being exposed and developed. With the aid of anisotropic reactive ion etching a nozzle orifice 11 with a diameter of 2 micron and a length of 1 micron is made in the silicon nitride layer and with use of dry and wet chemical KOH etching a cavity 13 with a diameter of 15 micron and a length of 400 micron is made in the silicon wafer 12.

The flow rate F of a medium or a liquid with viscosity h through an orifice (tube) with length L and diameter D for viscous flow at a pressure difference DP is given by the law of Poiseuille:

$$F_{Poiseuille} = pD^4 DP/128Lh.$$

A parabolic velocity pattern with a low velocity along the wall and a high velocity in the middle of the tube will settle in case the length of the tube L is larger than typical six times the diameter D. The mean velocity v of the medium or liquid is always given by $v = 4F/pD^2$.

In case the length L is of the order of the diameter D, the law of Poiseuille will change to the law of Stokes:

$$F_{Stokes} = D^3 DP/24h.$$

The parabolic velocity pattern will not be valid in this regime. Dagan et al., Chem.Eng.Svi., 38 (1983) 583-596 have proposed an interpolation formula for both regimes:

$$F_{Dagan} = D^3 DP/24h \ [1+16L/3pD]^{-1}.$$

At large velocities v the viscous regime will not be valid any more because another force/pressure is necessary for a kinetic (inertial) contribution to accelerate the medium or fluid to a velocity v. This pressure difference is given by $DP_{kin} = 0.5rv^2$, with r the mass density of the fluid v (cf. Law of Bernoulli).

An important insight according to the invention is that the total needed pressure $DP_{tot}$ is the sum of the viscous and the kinetic contribution:

$$DP_{tot} = DP_{vis} + DP_{kin} = 6hp[D+16L/3p]^{-1} \ v + 0.5 \ r \ v^2.$$

Typical for a waterbased fluid and for a thin orifice this means:

$$DP_{tot} \gg 18.000D^{-1} \ v + 500 \ v^2$$

( $L<D$, $h=10^{-3}$ poise, $r=1000$ kg/m$^3$, D in micron, v in m/s, DP in Pascal). At a $DP_{kin}$ of 4 bar (=$4x10^5$ Pascal) the maximum jet velocity will be 28 m/s. $DP_{vis}$ will be for this velocity 500.000 $D^{-1}$. In case D > 2 micron than $DP_{vis}$, < 2.5 bar. $DP_{tot}$ is then 4 + 2.5 = 6.5 bar, less than the maximum of 10 bar. However in case L/D > 2 then at D= 2 micron the needed pressure will surely exceed 10 bar.

Another important insight is that with a very thin orifice (L»D»micron) both in the viscous and in the kinetic regime all the fluid will leave the orifice as a jet with constant velocity v (no parabolic velocity distribution). Especially the kinetic energy of the jet will make that the jet will prolong its track before it breaks up in small droplets, which is particularly useful for Rayleigh break-up of the jet in droplets in air. Rayleigh droplets have a typical droplet size 1.6 times the diameter D of the out coming jet. The fabrication tolerance in the diameter D of the nozzle orifice is an essential factor in determining the amount of liquid (DV=4p $(1.6D/2)^3/3$) in a Rayleigh droplet. The United States FDA imposes a repeatability of 20% for 90% of the droplets and 25% for the remaining 10%. Only micro machining methods are capable of producing orifices with a tolerance less than 3% (= variation in DV< 10%). Also because micro machining is done in a sterile and particle free Clean Room environment also the effect of fouling of the nozzles due to particles and/or micro organisms is avoided.

Another important insight according to the invention is that for a very thin orifice(L»D»micron) the flow rate at relatively low pressures (3-10 bar) is mainly determined by the kinetic contribution, which means that viscosity of the fluid (medicine) has a minor role as long as L»D and $h<10^{-2}$ poise. Jetting (e.g.Rayleigh break-up) with a nozzle plate with a thickness less than 2 micron and orifices with a diameter between 0.4 and 10 micron at a pressure in which the contribution of the kinetic regime ($0.5rv^2$.) is larger than the contribution of the viscous regime ($6hp[D+16L/3p]^{-1}$.v) is therefore a very good method to deliver and dose medicines nearly independent of the viscosity of the medicine.

Another important insight according to the invention is that medicine (e.g. proteins and peptides) degradation is strongly diminished if such thin orifices are used at relatively low jetting pressures (<10 bar) with a minimum of shear in strength, time and length of the medicine in passing such an orifice.

Using the law of Stokes and Poiseuille (or Dagan) it is easily to calculate that the flow resistance of the 2 micron orifice 11 is still 5-10 times higher than the flow resistance of the cavity 13 with diameter 15 micron and length 400 micron. This means that the pressure/flow characteristics of this structure is still mainly determined by the 2 micron orifice.

In preference the thickness of the nozzle plate 10 for atomisation is less than six times the diameter of the nozzle orifice 11 and in preference less than one to two times the diameter in order to prevent the built up of a parabolic velocity distribution. The flow resistance may be further reduced through the manufacturing of tapering orifices although it is well known that the amount of tapering is very difficult to control precisely. In case the nozzle plate 10 has a thickness less than 2 micron it still has sufficient strength and it is not necessary to taper the orifices.

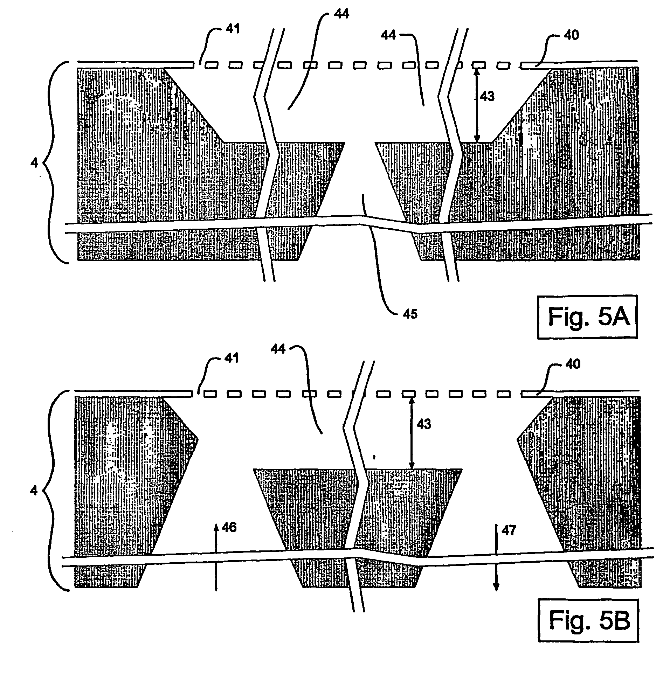

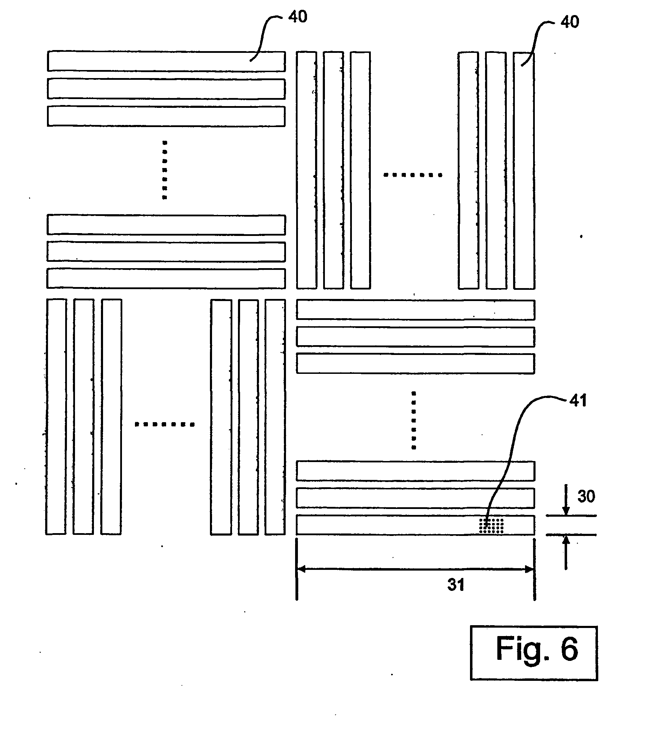

Nozzles can be used for as well atomisation and filtration. An embodiment of a nozzle with a nozzle orifice for atomisation or filtration 4 is shown in FIG. 5A, 5B and topview Fig. 6. The nozzle plate 40 comprises more nozzle orifices 41 placed next to each other in order to increase the throughput. The nozzle plate 40 with a thickness of 1 micron of low stress silicon nitride has a width of less than 250 micron 30 and a length of more than 300 micron 31. The maximum pressure strength of each nozzle plate 40 is well above 10 bar. A nozzle plate 40 with a width of 100 micron has a pressure strength well above 20 bar. A number of those nozzle plates 40 are closely packed with a mean distance less than 100 micron offering a large effective nozzle plate area as seen in topview FIG. 6.

The nozzle 4 comprises further at least one shallow flow channel 44 connected to the nozzle plate 40 with a mean depth of minimum 10 and of maximum 300 micron connected to the nozzle plate. This depth 43 is dependent on the size and number of the nozzle orifices 41 in the nozzle plate 40. The flow resistance of the flow channel 44 in the nozzle plate support should be at least one to ten times smaller than the flow resistance of the nozzle plate 40 itself. In case the total flow resistance of the nozzle plate support as defined by regions 44 and 45 is one to five times the flow resistance of the nozzle plate 40 a nice flow limitation has been constructed in case the nozzle plate 40 would disrupt. Alternatively two or more openings 46,47 can be provided in each nozzle plate to promote fluid flow and the removal of particles and air bubbles underneath the nozzle plate 40.

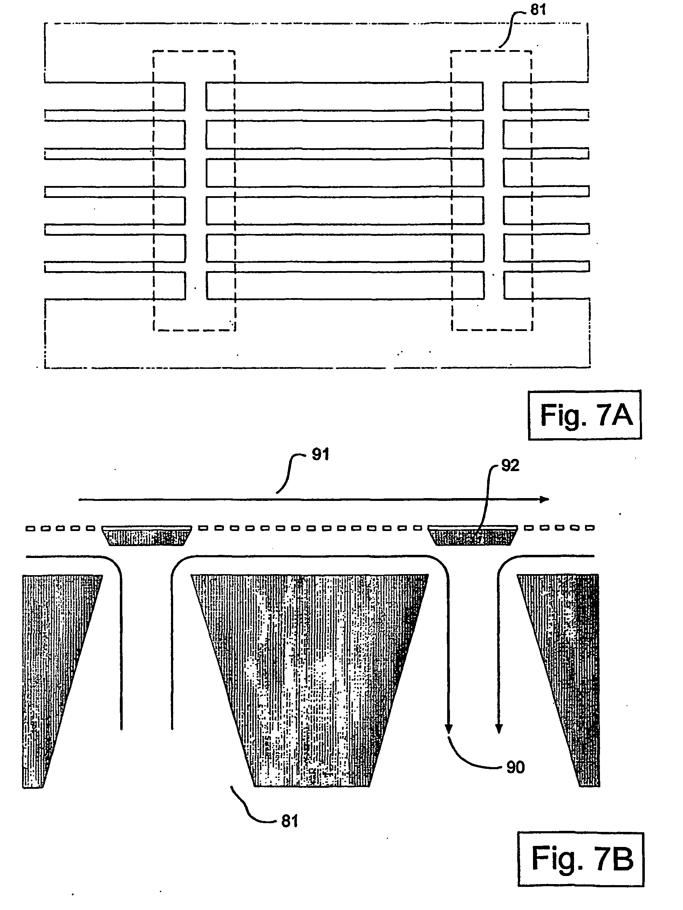

Cross-flow cleaning 90,91 on both sides of the nozzle plate is enhanced by the interconnection 81 in one or more directions of all nozzle plate support flow channels 44 (FIG. 7A,7B). Silicon bars 92 between the nozzle plates 40 may be provided for enhanced strength.

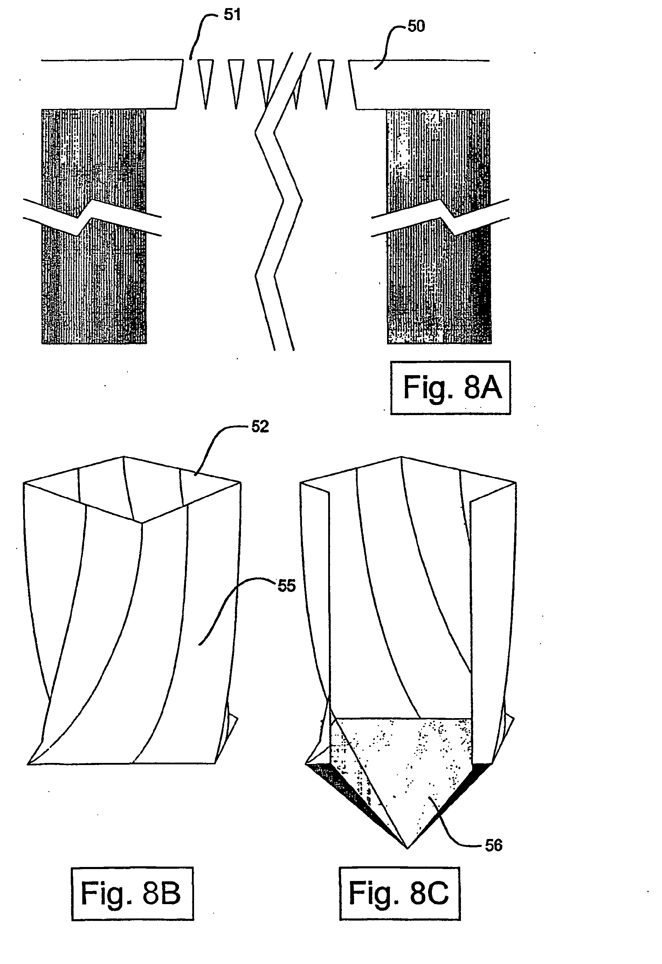

Subsequently the nozzle plate 50 may be chosen thicker than a few micron with corresponding tapering orifices 51 in order to reduce the flow resistance still further, shown in FIG. 8A. A good measure is also to make spiral grooves 55 in the nozzle orifice 51 to give the medium a rotational motion when leaving the orifice 51, shown in FIG. 8B. Anisotropic and directional etching techniques with $SF_6$ and $O_2$ at low bias voltage 10-40 eV make it possible to make such grooves in e.g. a <100> silicon wafer. The groove 55 will start at a defined rectangular orifice 52, the groove will turn and will stop turning as defined by the orientation of the <111> planes 56 shown in FIG. 8C.

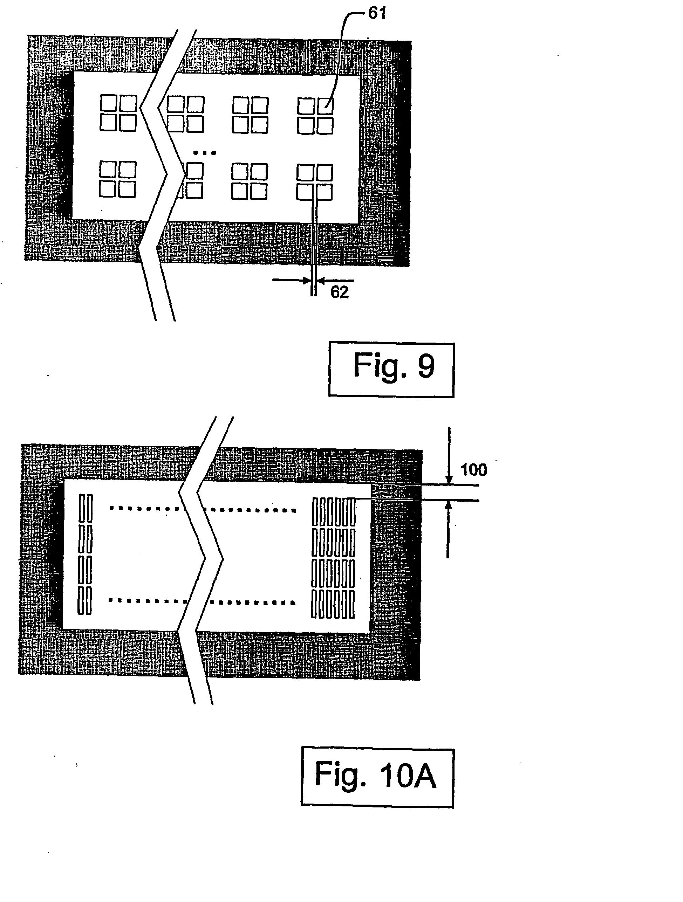

With preference a number of nozzle orifices 61 are placed very close together (FIG. 9), which improves flow rate, filtration and kinetic jetting behaviour, e.g. 2 or more nozzle orifices with a diameter of 2 micron may be separated with a mean distance less than 0.5 micron 62.

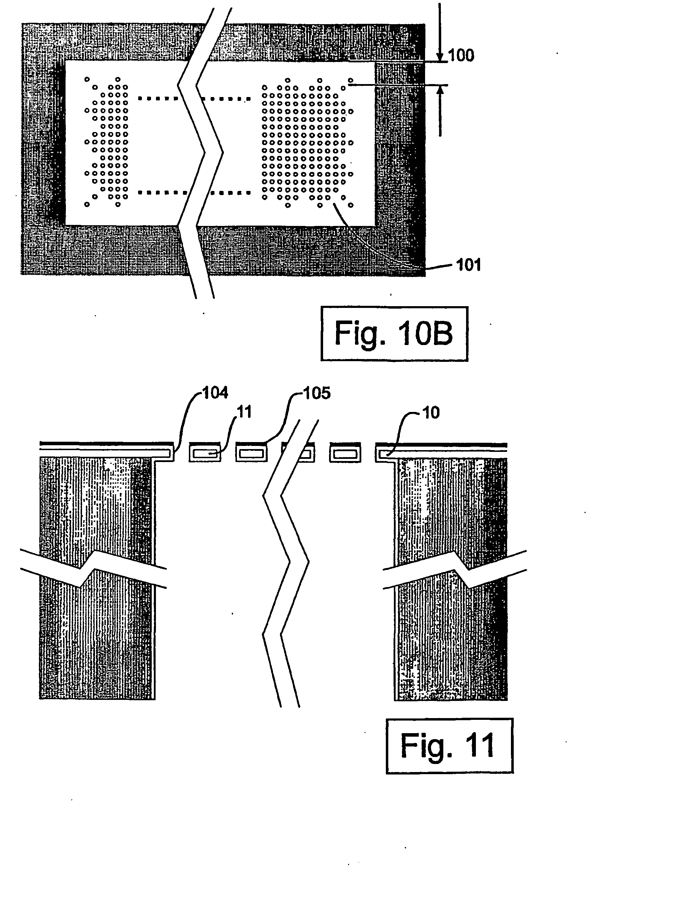

Nozzle plates can be made substantially stronger (up to 250%) when the nearest distance 100 between all nozzle orifices and the nozzle plate support is at least six times the thickness of the nozzle plate FIG. 10A, 10B. The pressure strength of the nozzle plate may be further increased with at least 50% when the orifices are placed in a triangular or rectangular pattern 101 with respect to a long side of the nozzle plate support. Preferential the orifices are slit shaped and placed parallel along the width of the nozzle plate support, FIG. 10B. An organic coating 104, in particular a parylene coating on the nozzle plate may further increase the pressure strength of the nozzle plate. Also a bacteria killing surface modification 105 may be applied, for example a silver coating, FIG. 11. A silicon nitride coating on the nozzle plate and the nozzle plate support may also be provided to make the whole structure inert for acid and caustic.

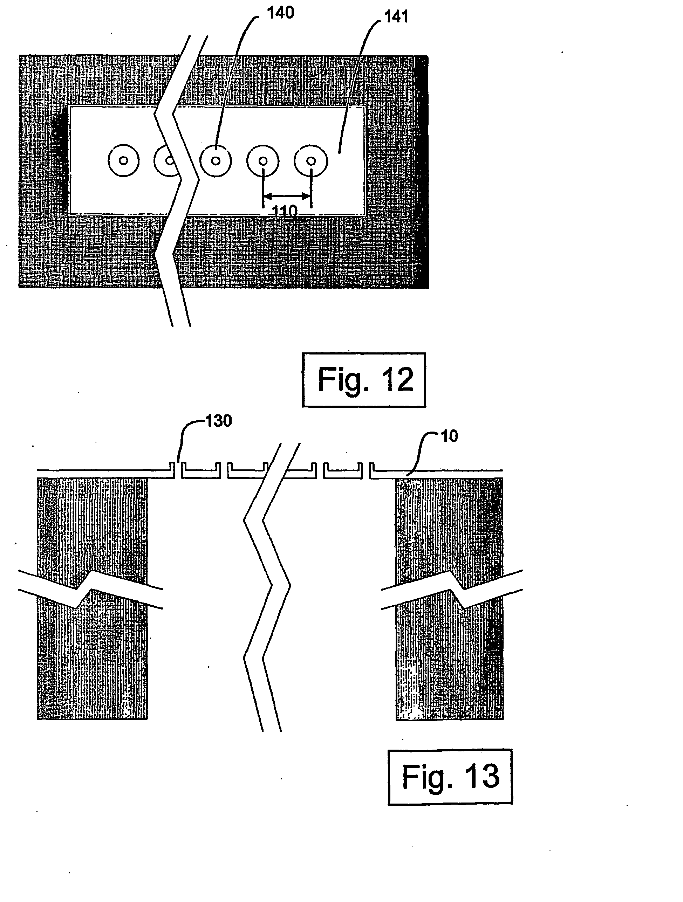

A next embodiment of a nozzle for atomisation is shown in FIG. 12,13 and 14. The nozzle plate 10 with a thickness of 1 micron comprises circular orifices with a diameter of 0.8 micron. The distance between any of two orifices of the nozzle plate is larger than five times 110 the nozzle diameter in order to prevent recombination of droplets formed of nozzles next to each other.

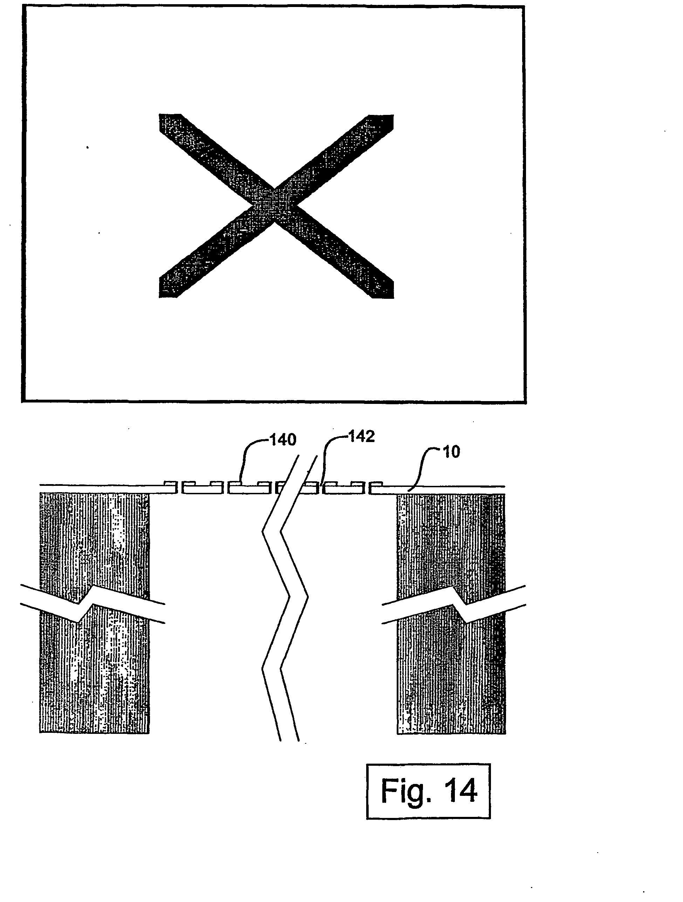

FIG. 13 shows a cross section of a nozzle plate wherein the orifices are slightly protruding out 130 of the surface (0.1 - 2 micron) of the nozzle plate 10. This measure is particular useful if the nozzle plate is used for jetting of a spray or emulsification because it prevents that the droplets will adhere to the surface of the nozzle plate just next to the nozzle orifice, resulting in to large droplet formation. In case of atomisation (FIG. 14) it is particular useful to make a small area (e.g. 2-5 micron radius around the nozzle) hydrophobic 140 to prevent droplet attachment/smearing and an unwanted increase of the jet diameter (confinement of the jet).

Preferential the next surrounding area 141 should be made hydrophilic in order to drain a too large formed droplet that might inhibit jetting behaviour along the hydrophilic coated part of the nozzle plate. Good results have been obtained with a micro porous hydrophilic coating (PolyVinyl Pyrolidone/ PolyEther Sulphone) with a thickness of a few micron on area 141.

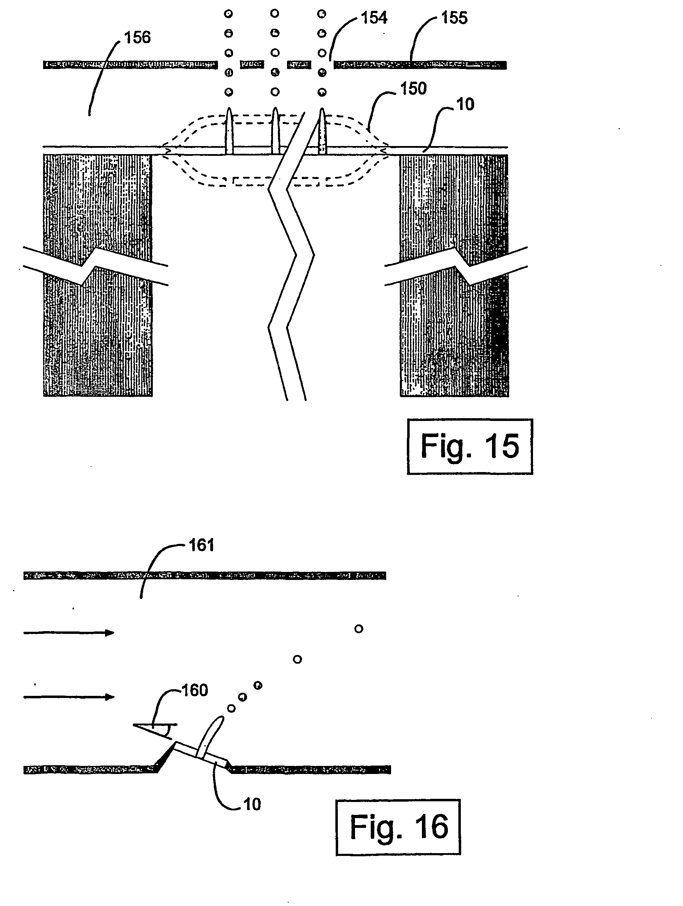

Alternatively capillary forces may be used to drain the droplet through e.g. the provision of a drain channel 156 with a height of e.g. 2 to 50 micron between the nozzle plate and an e.g. parallel placed drain plate 155 with an orifice 154 larger (e.g. 5 micron) than the jetting orifice (e.g. 1 micron), see FIG.15. The jet will normally first pass the jetting orifice and next the larger orifice without fluid contact with the drain plate, but as soon as a droplet is formed that touches the drain plate it will be drained through the drain channel. A hydrophilic inner surface 142 of the nozzles of the nozzle plate is a good measure in order to suppress a large Pascal Pressure with at least 50%.

Jetting may be enhanced by using a piezoelectric actuator at a frequency between 100 kHz and 3 Mhz. Jetting may also be enhanced using the eigenresonance frequencies of the nozzle plate. This frequency should match the value of the initial jet velocity divided by two to two hundred times the diameter of the nozzle, typically a value between 50 kHz en 5 MHz. The eigen-resonance frequency is mainly determined by the mass and a fortiori lateral dimensions of the free hanging nozzle plate (typical 1x10x10micron to 4x250x2000 micron), the rigidity of and the tensile pre-stress in the nozzle plate (typical $10^6$ to $10^9$ Pascal). A vibrating nozzle plate 150 is shown in FIG. 15

Measures to prevent droplet coalescence include: to charge the droplets during droplet formation with an external voltage, or by friction (tribocharging) of the fluid in the nozzle plate device, or by friction of the droplets with the air. An electrical connection (short-circuit) between the patient and the atomising device may be necessary (patient serves as an earth electrode). Further measures include placing the nozzle plate at an angle 160 between 10° and 90° in a cross flow channel 161, particularly useful for Rayleigh break-up (FIG. 16) or co-flow of air or another medium through separate nozzles orifices.

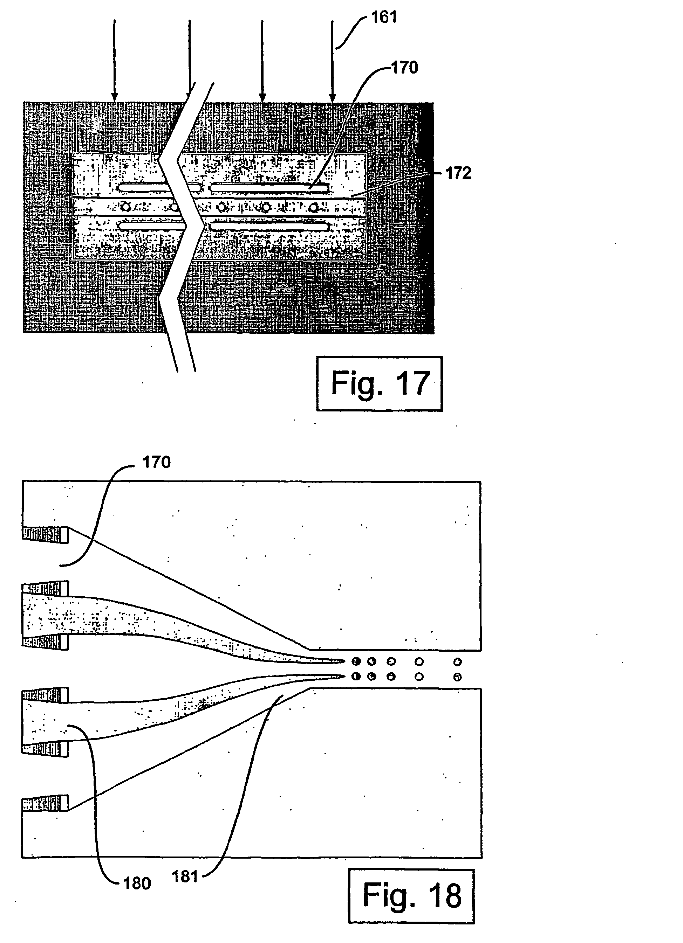

Good results have been obtained with a nozzle plate design (FIG. 17) in which the nozzles for atomisation are lined up and the nozzles for co-flow 170 are lined up perpendicular to the cross flow channel. The nozzles for the liquid and the nozzles for the air have separated 172 supply channels. For emulsification it is particularly useful to place the nozzle plate in a dead-end configuration in the channel, which channel 181 narrows significantly at the downstream side. With relatively large nozzle orifices it is then possible to make very small emulsion droplets or gas bubbles (FIG. 18) at least 2 -10 times smaller than the diameter of the smallest nozzles 180. Double emulsions (o/w/o or w/o/w) can advantageously be made with this device, because small emulsion droplets can easily pass the larger orifices without coalescense.

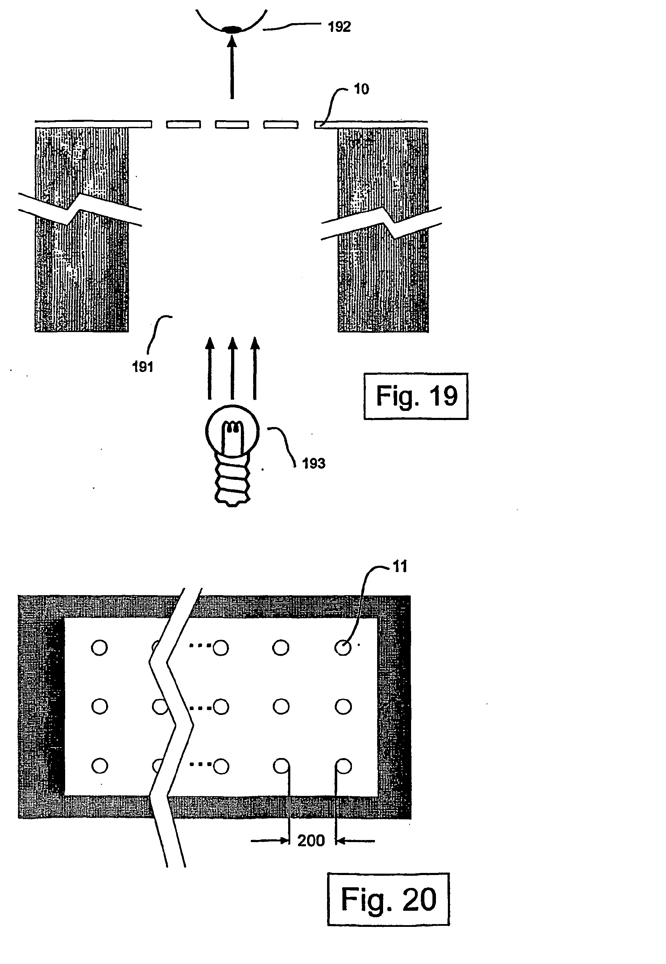

FIG. 19 and 20 show an embodiment of a filter means or nozzle for retaining particles. The nozzle plate support body is made out of one silicon <110> wafer. With this type of crystal orientation it is possible to make perpendicular openings in the support by use of wet (KOH) etching. When the width between two walls of the nozzle plate support body 191 is chosen small (e.g. 20 micron) it is possible to make a high flow resistance in the nozzle cavity. For filtration purposes it has proven to be useful to make an open porosity higher than 30%, not only because of the high flux, but also because it is then relatively easy to back-flush the membrane to prevent irreversible fouling.

The nozzle plate may also be used for retaining and subsequent microscopic observation 192 of these particles, e.g. bacteria's, yeast cell's, blood cell's, etc. Fluorescent dyes may be used to simplify and identify specific species of the micro-organisms on the filter. Silicon nitride and other inorganic nozzle plate materials have the advantage in contrast to many organic polymeric materials that there is virtually no auto-fluorescence signal from the material itself. In some cases it is convenient to place the nozzle orifices further apart, in order to isolate the micro-organisms from each other for a more easy recognition and enumeration.

Very useful nozzle plates for this purpose are **characterised in that** the spacing 200 between the nozzle orifices is minimum three and maximum thirty times the diameter of the nozzle orifices.

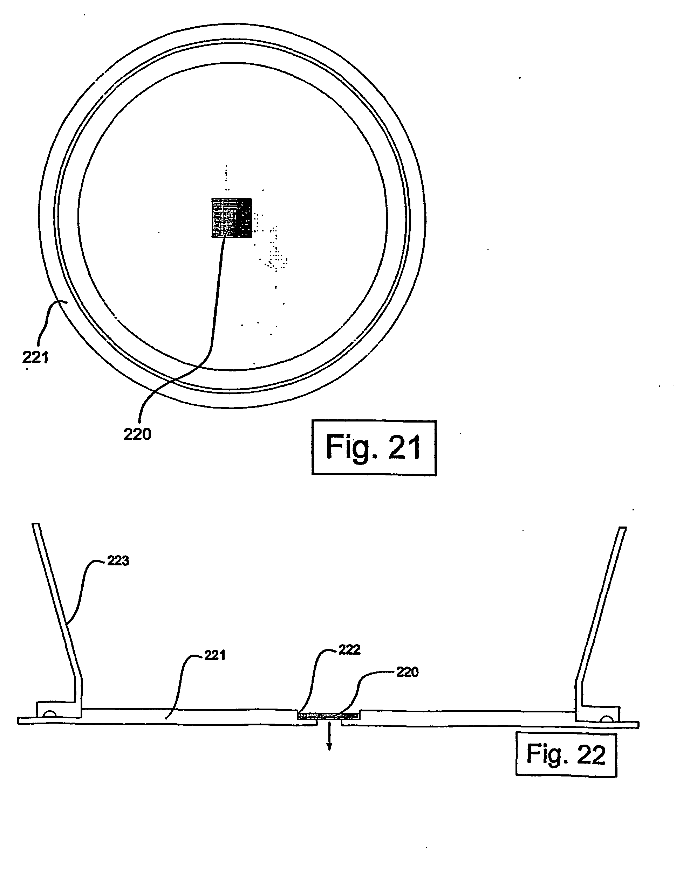

Filter means or nozzles may be used for disposable filtration applications, with preference small nozzle plates 220 (e.g. 5x5mm) are embedded in a ring shaped support 221 (e.g. ABS plastic discs) with outer dimension of e.g. 1.0, 2.5 and 5 cm in diameter and ready to use in standardised commercial filtration holders. With preference the nozzle plates are countersunk 222 with a depth of 10 to 500 micron in the ring shaped support to prevent contamination, to facilitate packaging and mechanical rupture of the nozzle plate (FIG. 22).

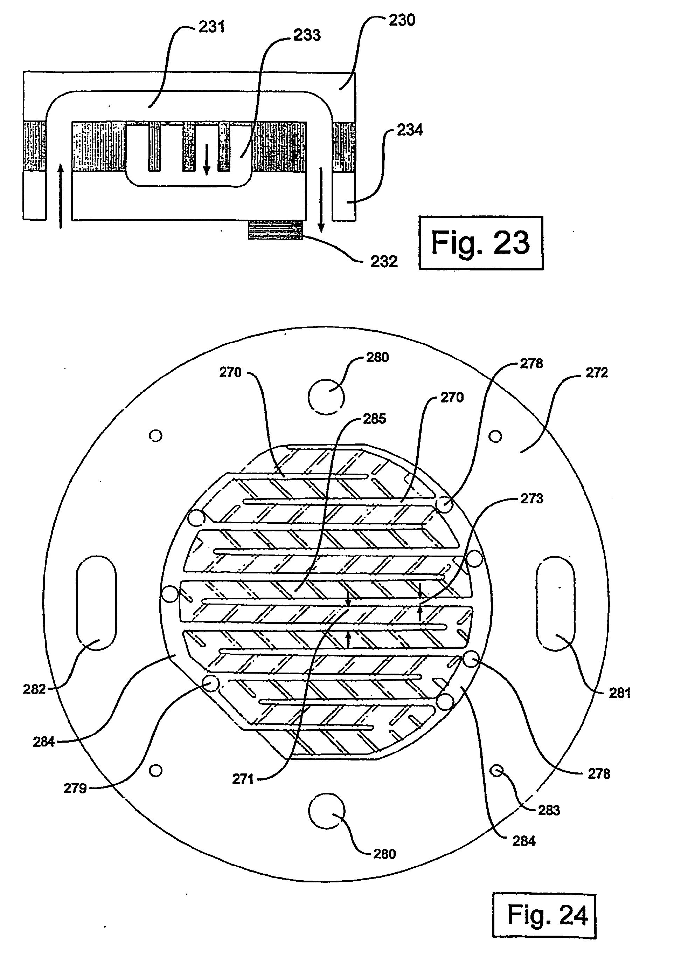

For reusable application an optic transparent cover slip 230 is placed over the nozzle plate in such a way that a cross-flow channel 231 with a depth of 50 to 500 micron exists between the nozzle plate and the cover plate (FIG. 23). With preference the cover plate is a glass like material that is anodically bonded to the nozzle plate or the nozzle plate support body at elevated temperature (300-400°C) at a voltage between 500 and 1500 V. Cleaning and reuse of this device is facilitated 232 by using ultrasound with a frequency between 100 kHz and 1 MHz. A liquid handling board 234 can be made in glass (with a preferred thickness between 0.5 and 11 mm) for supply of liquid to and from the nozzle plate. By using an anodic bond between the nozzle plate or the nozzle plate support body and the liquid handling board, glass can be used as a liquid handling board for applications in which the required pressures are higher than 0.8 bar.

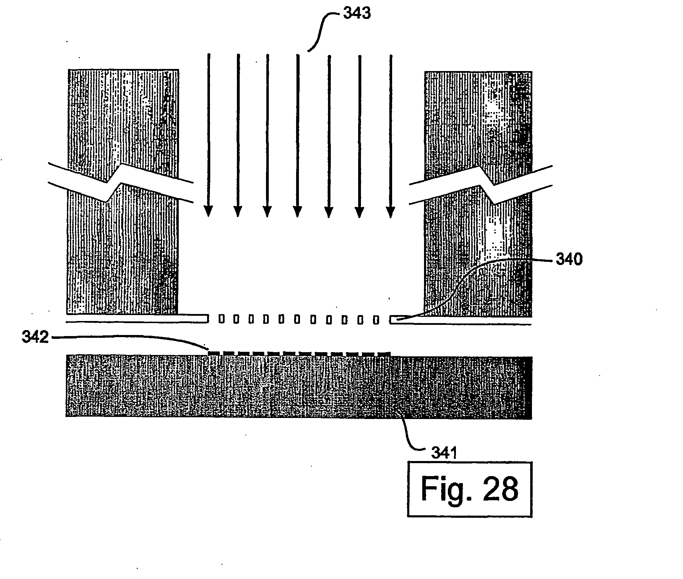

With preference the nozzle plate support body has cavities 233 with at least the same size as the nozzle plate. It is then possible to use a microscope 192 with a light source that projects light 193 first through the nozzle support and next on the nozzle plate. Most microscopes with phase contrast mode work in this manner. FIG. 28 shows a nozzle plate 340 that can also be used for the deposition (stencilling) 343 of isolated material spots 342 on a substrate 341 with feature sizes determined by the lay out of the nozzle plate. <110> silicon is a good support material for the nozzle plate for these purposes.

Large nozzle plates with an outer circular diameter of e.g. 2, 3, 4, 6 and 8 inches may be used for micro filtration applications like yeast cell filtration and clarification of beer and other beverages. Sterile filtration of milk and other dairy products is also possible with pore sizes between 5 and 0.22 micron. With a pore size of 0.8 micron it has been tested that a log reduction of 5 to 6 of micro-organisms in milk is well achievable in combination with back-pulse (pulsed permeate flow reversal) technology. Typical flow rates are 1000 -2000 $l/m^2$/hour at low trans-membrane pressures (0.03 -0.1 bar) with a back-pulse rate of 0.01 - 5 Hz.

The flow rate can be strongly increased (4000-20.000 $l/m^2$/hour) using ultrasound in a broad frequency spectrum between 100Hz - 1 MHz. Preferably a frequency is used under 15 kHz or above 50 kHz in order to suppress the cavitation forces that might disrupt the nozzle plates between 15 kHz and 50 kHz. The ultrasound inhibits the forming of a dense cake layer just before the nozzle plate. Alternatively the performance for jetting, filtering, foaming and emulsification may be improved by moving the nozzle plate tangential and/or orthogonal to the fluid in contact with the nozzle plate with an actuator with an amplitude of 0.1 to 100 micron and a frequency of 10 Hz - 10 MHz.

In a special embodiment the nozzle plates or nozzle plate support bodies are bonded to a glass plate in which flow channels 270,284 have been made with the use of grinding or powder blasting (FIG. 24, 25 and 26). Glass plates of type borosilicate have the advantage that they are very flat, have nearly the same thermal expansion coefficient $4.10^{-6}°C$ as nozzle plates with a silicon support. Anodic bonding results in a bond inert for acid, caustic and oxidizing chemicals. The flow channels may be used for permeate flow or alternatively for cross-flow.

Preferably the flow channels for cross-flow are placed in comb like structures which taper in length and/or in height. The comb structure has the advantage that the total pressure drop over the shallow channel area 285, the comb teethes 270, the inlet 278 and outlet 279 (through the glass plate) can be kept low (less than 100mBar), while the cross flow speed at the nozzle plate surface (at the shallow channel area 285) is yet high enough (more than 0.1m/s) for the enhancement of continuous removal of particles and yeast cells during filtration. The distance 271 between the teethes 270 of each comb are preferably 0.5 - 5 cm, with a depth of 1 - 5 mm, a width of 1 - 5 mm and a length depending on the outer circular diameter.

The tapering of the depth 274 is preferably 10° to 40°. The width 273 is preferably tapering 10-40% per cm length of the channel. In particular when powder blasting is used to manufacture the channels in the glass plate, there is a triangular shape of the channels with a relation of the width of the channel and the depth if 1.2. The tapering is meant for a good redistribution of the fluid from the incoming channel to the outgoing channel in such a way that the pressure distribution along a single tooth of the cross flow channels is homogeneous while the fluid velocity never reaches zero to avoid hygienic failure. The pressure drop over every single tooth is equal by varying the width and the depth of the tooth. The mean cross flow height between the glass plate and the nozzle plate 276 in the shallow channel area is preferably between 0.1 and 1 mm. As well the cross-flow side as the permeate side may be bonded to a glass plate, also one glass plate may be bonded on both sides with a nozzle plate device.

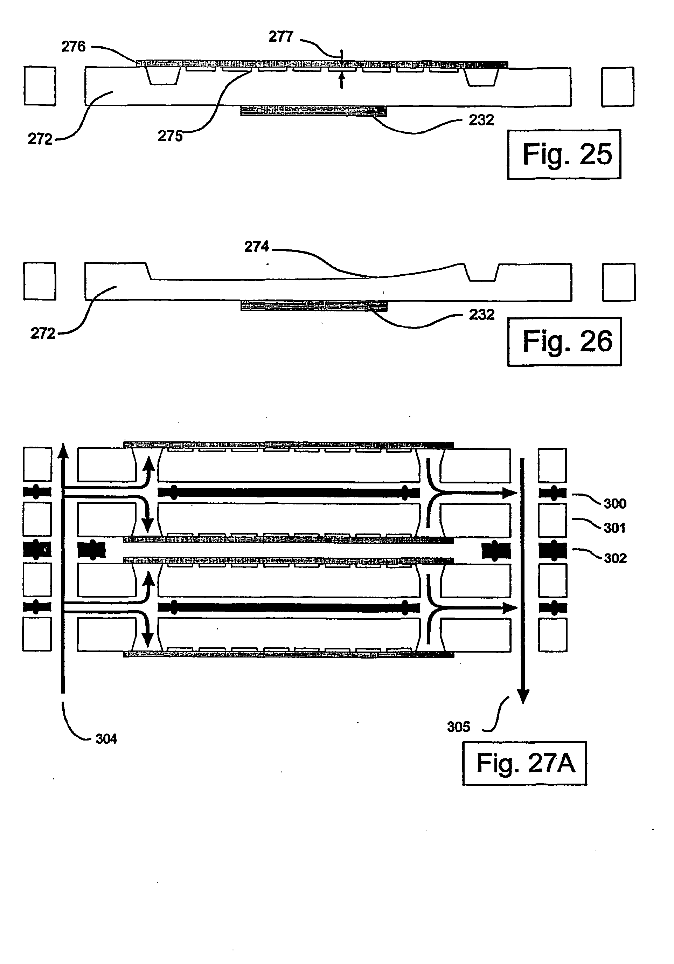

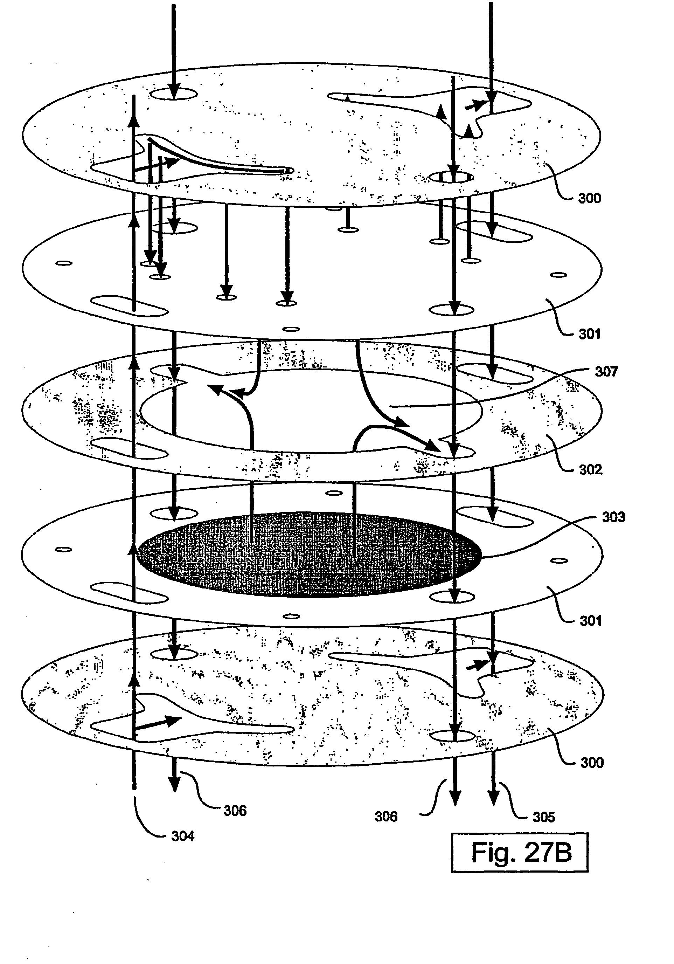

With preference the glass plate is being used for a filtration module, where a larger filtration capacity is achieved by placing a number of nozzle plate devices 301 with spacer structures 300, 302 in a stack (plate and frame module with mirror placing of the glass plate 301 and the nozzle plates 303, FIG. 27A, 27B. The glass plate acts in this module also as tubing for the cross flow inlet 281, 304, cross flow outlet 282, 305 and permeate collection 280, 306, 307. Furthermore, the glass plate may contain holes 283 for easy positioning of the glass plate and the spacer structures.

Filtration characteristics may also be enhanced by using rotating nozzle plates with respect to the medium in a module. A piezo transducer for ultrasound can be placed on the back side (non powder blasted side) of each glass plate. A typical longitudinal resonance frequency of a glass plate with a thickness of 10 mm is 250 kHz. The ultrasound may be used either for enhancement of the flow rate during filtration or for cleaning of the nozzle plates after or during the filtration cycle. Of course cleaning after filtration with ultrasound is accelerated using proper chemicals (acid/caustic/enzymes etc.). Normal chemical cleaning procedures as used for micro and ultra filtration membranes can herewith be reduced from 1-2 hours back to 10 seconds - 5 minutes. Cross-flow cleaning on both sides of the nozzle plate is enhanced by the interconnection in one or more directions of all nozzle support openings.

Nozzle plates made with a silicon support can be made chemically inert for caustic media by providing a thin LPCVD grown silicon nitride coating with a typical thickness between 0.01 and 1 micron. Other organic and inorganic coatings like e.g. $Al_2O_3$, $TiO_2$, $ZrO_2$, $ZrO_2/Si_3N_4$ may be applied to alter the Zeta potential and/or the wetting properties of the nozzle plate to improve filtration characteristics. Other coatings may also be applied to promote anti-fouling like $TiO_2$, PTFE, self assembling monolayers (SAM, e.g. based on nitryls, disulfides or thiols) or long polymer chains (e.g. polyethylene glycol) coupled with an end- or side- group to the nozzle plate. Dense sol/gel coatings or gas permeation layers like Pd, PdAg may also be applied over and in the nozzle orifices to make ultra-filtration and gas filtration membranes.

An important insight according to the invention is that the combination of nozzle plates, back-pulse technology and ultrasound has proven to be very powerful for the enhancement of flow rate and the prevention of irreversible fouling. Without ultrasound a typical clarification run for beer is 4-8 hours, with ultrasound dosed at intervals of 10 minutes for a few seconds the run can be extended to 4-8 days without the need of chemical cleaning procedures.

Back pulsing for a very short time 10-50 ms at regular intervals 0.01 - 5 Hz during cross-flow filtration at low trans-membrane pressure will lift the cake layer from the nozzle plate and will inject it higher in the cross flow channel where the fluid velocity is sufficient high to take it further away.

Back pulsers are also very suitable to use for up-concentration of samples for the detection and counting of food spoiling or pathogenic micro-organisms, e.g. lacto bacillus, E-coli and legionella. After the up-concentration all micro-organisms are present on the nozzle plate and can be processed for e.g. microscopic observation and PCR amplification. Small nozzle plates of e.g. 4x4 mm can be put easily with a clean and sterile pincer in a small PCR-cup.

The nozzle plate can also be provided with an immuno binding (or elisa coupling) agent for the selective binding of certain species direct to the nozzle plate during filtration, especially when cross-flow techniques are used for up-concentration of the sample. Magnetic layers may also be deposited for the attraction of immuno magnetic beads.

Metallic layers may also be provided on the nozzle plates for e.g. optic non-transparency, non quenching or electrolysis applications, improvement of filtration under the appliance of a small voltage difference between the fluid and the nozzle plate, or the annihilation (electroporation) of microorganisms under the appliance of a high voltage pulse. Platinum may be deposited in electrical resistor strips on the nozzle plate for heating purposes. Also a bacteria killing surface modification may be applied, for example a silver coating.

Piezo materials may also be applied for direct vibration of the nozzle plates or for the detection of bending of the nozzle plates for pressure registration. The intensity and the frequency of the back-pulsers may also be regulated by the registration of the nozzle plate trans membrane pressure. The trans membrane pressure will normally increase if there is a built up of a cake layer for the nozzle plate.

Nozzle plates can be made in various ways according to the invention. A reinforced micro-machined polymeric nozzle plate is made by

- depositing a first layer of a photosensitive material, for example negative resist polyimide (Durimide 7510) on a flat and smooth substrate
- exposing the first layer to a suitable light source through a mask (or a laser interference pattern) with a nozzle pattern
- developing and if necessary curing the first layer
- depositing a second layer of a photosensitive material onto the first layer
- exposing the second layer to a suitable light source through a mask with a nozzle support structure
- developing and if necessary curing the second layer
- releasing the thus obtained nozzle plate from the substrate

Another method of making a micro-machined polymeric nozzle plate, comprises the following steps

- depositing a first layer of a photosensitive material on a flat and smooth substrate
- exposing the first layer to a suitable light source through a mask (or laser interference) with a nozzle pattern
- developing the first layer
- anisotropically etching the nozzle pattern to a certain depth, typically 1 to 5 micron, in the substrate
- depositing a second layer of a photosensitive material onto the substrate
- exposing the second layer to a suitable light source through a mask with a nozzle support structure
- developing the second layer
- etching anisotropically the nozzle support structure to a certain depth, typically 5 to 500 micron, in the substrate
- electroforming a master mould from the substrate if necessary or using the substrate itself as a master mould
- if necessary depositing a release agent (teflon) on the master mould
- placing a thin sheet of thermoplastic polymer with a typical thickness between 5 and 50 micron onto the master mould
- placing a second (flat) substrate with a release agent on the polymeric sheet
- pressing the two substrates to each other with a substantial load at a temperature well above the glass transition temperature of the polymeric sheet if necessary under reduced atmospheric conditions for a short period
- releasing the thus formed polymeric nozzle plate from the substrates at a temperature well below the glass transition temperature

A reinforced micro-machined electroformed nozzle plate is made by

- depositing a conductive layer on a flat and smooth electrically insulating substrate
- depositing a first layer of a photosensitive material on the conductive layer
- exposing the first layer to a suitable light source through a mask with a nozzle support pattern
- developing the first layer
- etching the conductive layer with a suitable chemical etchant
- removing the first layer
- depositing a second layer of a photosensitive material with a thickness of at least 2 micron onto the substrate

- exposing the second layer to a suitable light source through a mask with a nozzle device
- developing the second layer such that the remaining resist layer is not in contact with the conductive layer
- putting the substrate in a suitable electroforming bath using the conductive layer as a cathode
- stopping the electroforming process as soon as the electroformed layer has reached substantially at least one or more parts of the remaining resist layer
- releasing the thus electroformed nozzle plate.

Another method of making a micro-machined nozzle plate device comprises the following steps

- depositing a first layer of a photosensitive material on a flat and smooth substrate, said substrate being covered at both sides with a thin membrane layer
- exposing the first layer to a suitable light source through a mask with a nozzle support pattern
- developing the first layer
- etching the nozzle support pattern in the membrane layer on one side of the substrate and further
- etching chemically the nozzle support pattern through the substrate stopping at a distance of 5 to 100 micron of the membrane layer at the other side of the substrate
- depositing a second layer of a photosensitive material onto the other membrane layer of the substrate
- exposing the second layer to a suitable light source through a mask (or laser interference) with a nozzle plate structure
- developing the second layer
- etching the nozzle plate structure in the membrane layer
- etching through the nozzles part of the nozzle support structure such that the nearest distance between the nozzles and the nozzle support structure is at least twice the nozzle diameter

Nozzle plates according to the invention may also be used for the extrusion of very viscous media like macromolecular solutions, gel-like solutions and protein-rich media, and for micro structuring of food and pharmaceutical products like e.g. synthetic meat (fibres). Nozzle plates according to the invention may also used for micro-array and micro-titration applications, to make double emulsions and to apply them in bio-capsules because of the small diffusion length of the short nozzle orifice.

## Claims

1. Device (1) for filtration and/or emulsification of a fluid, comprising a nozzle plate (10,15,40,50) and a nozzle plate support body (12,17), said nozzle plate support body carrying said nozzle plate (10,15) at a main surface side, wherein said nozzle plate has a thickness of less than 2 micron and comprises a plurality of nozzle orifices (11,16,51,61) extending over said thickness through said nozzle plate, wherein said support body comprises a cavity (13) and said plurality of nozzle orifices are jointly in fluid communication with said cavity, and wherein a nozzle orifice within said plurality of nozzle orifices has a length which is less than six times its diameter, and in particular is shorter than its diameter, **characterized in that** said nozzle orifices of said plurality of nozzle orifices are arranged in a first zone (61,101) of said nozzle plate, **in that** said nozzle plate comprises a second zone (100) adjacent said first zone which is at least substantially free of any nozzle orifice, **in that** said cavity (13) extends underneath both said first zone and said second zone of said nozzle plate, and **in that** said second zone has a width which is at least a number of times as large as said thickness of said nozzle plate.

2. The device according to claim 1, wherein at least one orifice (11,16) in said nozzle plate (10,15) has a diameter between 0.4 and 10 micron.

3. The device according to claim 1 or 2, wherein a mutual spacing (62) between an orifice and an adjacent orifice is between three and thirty times a diameter of said orifices.

4. The device according to anyone of the preceding claims, wherein said nozzle plate comprises a number of substantially identical orifices which are spaced apart over a mutual distance (62) which is between 3 and 30 times, particularly between 3 and 10 times, their diameter.

5. The device according to anyone of the preceding claims, wherein said nozzle plate has a bare surface and wherein said orifice protrudes slightly out of said bare surface of said nozzle plate.

6. The device according to anyone of the preceding claims, wherein, at said main surface, said cavity has a cross-section with a width of less than 250 micron, particularly less than 100 micron.

7. The device according to claim 6 wherein said cross-section has a length of more than 300 micron.

8. The device according to anyone of the preceding claims, wherein said support body comprises a silicon substrate, particularly from <110> silicon wafer.

9. The device according to claim 8, **characterized in that** said nozzle plate is formed from a separate layer provided on said substrate, particularly a silicon nitride layer or a silicon carbide layer.

10. The device according to anyone of the preceding claims, wherein a glass substrate is bonded to said nozzle plate and wherein said glass substrate comprises at least one flow channel which is in open communication with said cavity in said support body supporting said nozzle plate.

11. The device according to anyone of the preceding claims, wherein said support body is suspended in a ring shaped support frame which is adapted to respective dimensions of a commercially available filter holder.

12. The device according to claim 11, wherein said nozzle plate is countersunk to a depth of between 10 and 500 micron in the ring shaped support frame.

13. The device according to anyone of the preceding claims, wherein an optic transparent cover plate is placed over the nozzle plate and wherein a flow channel with a depth of 50 to 500 micron is present between the nozzle plate and the cover plate.

14. The device according to anyone of the preceding claims, wherein nozzle plate is provided with a metallic layer facilitating optic non-transparency, non quenching, electrolysis or electric heating applications.

15. The device according to anyone of the preceding claims, wherein the nozzle plate and the nozzle plate support body are covered by a caustic resistant coating, particularly a silicon nitride coating.

16. The device according to anyone of the preceding claims, wherein the nozzle plate is provided with a piezo-electric actuator device.

17. The device according to anyone of the preceding claims, wherein the nozzle plate is provided with an immuno binding agent (or Elisa coupling), in particular a magnetic layer enabling the coupling of immuno magnetic beads.

18. The device according to anyone of the preceding claims, wherein the nozzle plate is provided with a sol/gel ultra-filtration coating or a gas permeation layer comprising palladium.

19. Method of making small shadow patterns on a substrate by using the device as claimed in anyone of claims 1-18.

20. A method of filtering beer or milk by using the device as claimed in anyone of claims 1-18.

21. A method of foaming or emulsifying a first fluid by using the device as claimed in anyone of claims 1-18, wherein said cavity carries a first fluid which is ejected through *said first zone of orifices* at said main surface and wherein said nozzle plate is brought into contact with a second fluid at said main surface.

22. The method according to claim 21, wherein said nozzle plate is moved tangentially and/or orthogonally with respect to said second fluid in contact with the nozzle plate.

23. The method according to claim 22, wherein a double emulsions is formed by using said nozzle plate.

**Patentansprüche**

1. Vorrichtung (1) zum Filtrieren und/oder Emulgieren eines Fluids, umfassend eine Düsenplatte (10, 15, 40, 50) und einen Düsenplattentragkörper (12, 17), der die Düsenplatte (10, 15) an einer Hauptflächenseite trägt, wobei die Düsenplatte eine Dicke von weniger als 2 Mikron aufweist und eine Mehrzahl von Düsenöffnungen (11, 16, 51, 61) umfasst, die sich über die Dicke durch die Düsenplatte hindurcherstrecken, wobei der Tragkörper einen Hohlraum (13) umfasst und die Düsenöffnungen mit dem Hohlraum gemeinsam in leitender Verbindung stehen, und wobei eine Düsenöffnung aus der Mehrzahl von Düsenöffnungen eine Länge aufweist, die geringer als das sechsfache ihres Durchmessers ist, insbesondere kürzer ist, als ihr Durchmesser, **dadurch gekennzeichnet, dass** die Düsenöffnungen der Mehrzahl von Düsenöffnungen in einem ersten Bereich (61, 101) der Düsenplatte angeordnet sind, dass die Düsenplatte einen zweiten Bereich (100) aufweist, benachbart zum ersten Bereich, der im Wesentlichen frei von jeglichen Düsenöffnungen ist, und dass sich der Hohlraum (13) unterhalb des ersten und des zweiten Bereiches der Düsenplatte erstreckt, und dass der zweite Bereich eine Breite aufweist, die wenigstens ein Mehrfaches größer ist, als die Dicke der Düsenplatte.

2.  Vorrichtung nach Anspruch 1, wobei die wenigstens eine Öffnung (11, 16) in der Düsenplatte (10, 15) einen Durchmesser zwischen 0,4 und 10 Mikron aufweist.

3.  Vorrichtung nach Anspruch 1 oder 2, wobei der gegenseitige Abstand (62) zwischen einer Öffnung und einer benachbarten Öffnung zwischen dem dreifachen und dem dreißigfachen eines Durchmessers der Öffnung ist.

4.  Vorrichtung nach einem der vorausgegangenen Ansprüche, wobei die Düsenplatte eine Anzahl von im Wesentlichen identischen Öffnungen umfasst, die einen gegenseitigen Abstand (62) aufweisen, der zwischen dem drei- und dem dreißigfachen liegt, insbesondere zwischen dem drei- und dem zehnfachen ihrer Durchmesser.

5.  Vorrichtung nach einem der vorausgegangenen Ansprüche, wobei die Düsenplatte eine nackte Fläche aufweist, und wobei die Öffnung geringfügig über die nackte Fläche der Düsenplatte hinausragt.

6.  Vorrichtung nach einem der vorausgegangenen Ansprüche, wobei der Hohlraum an der Hauptfläche einen Querschnitt mit einer Weite von weniger als 250 Mikron aufweist, insbesondere weniger als 100 Mikron.

7.  Vorrichtung nach Anspruch 6, wobei der Querschnitt eine Länge von mehr als 300 Mikron aufweist.

8.  Vorrichtung nach einem der vorausgegangenen Ansprüche, wobei der Tragkörper ein Silikonsubstrat umfasst, insbesondere aus einer <110> Silikonwafer.

9.  Vorrichtung nach Anspruch 8, **dadurch gekennzeichnet, dass** die Düsenplatte aus einer separaten Lage gebildet ist, die auf dem Substrat angeordnet ist, insbesondere einer Silikonnitridlage oder eine Silikoncarbidlage.

10. Vorrichtung nach einem der vorausgegangenen Ansprüche, wobei ein Glassubstrat an die Düsenplatte angebunden ist, und wobei das Glassubstrat wenigstens einen Strömungskanal aufweist, der mit dem Hohlraum im Tragkörper, der die Düsenplatte trägt, in leitender Verbindung steht.

11. Vorrichtung nach einem der vorausgegangenen Ansprüche, wobei der Tragkörper in einem ringförmigen Tragrahmen aufgehängt ist, der durch entsprechende Abmessungen eines im Handel erhältlichen Filterhalters gestaltet ist.

12. Vorrichtung nach Anspruch 12, wobei die Düsenplatte auf eine Tiefe von zwischen 10 und 500 Mikron im ringförmigen Tragrahmen versenkt ist.

13. Vorrichtung nach einem der vorausgegangenen Ansprüche, wobei eine optisch transparente Deckplatte auf die Düsenplatte aufgelegt ist, und wobei ein Strömungskanal mit einer Tiefe von 50 bis 500 Mikron zwischen der Düsenplatte und der Deckplatte vorgesehen ist.

14. Vorrichtung nach einem der vorausgegangenen Ansprüche, wobei die Düsenplatte mit einer metallischen Schicht versehen ist, die eine optisch nicht transparente, non quenching Elektrolyse oder elektrische Heizanwendungen ermöglicht.

15. Vorrichtung nach einem der vorausgegangenen Ansprüche, wobei die Düsenplatte und der Düsenplattentragkörper von einer kaustisch resistenten Beschichtung bedeckt ist, insbesondere von einer Siliziumnitridbeschichtung.

16. Vorrichtung nach einem der vorausgegangenen Ansprüche, wobei die Düsenplatte mit einer piezoelektrischen Aktuatorvorrichtung versehen ist.

**17.** Vorrichtung nach einem der vorausgegangenen Ansprüche, wobei die Düsenplatte versehen ist mit einem immunen Bindemittel (oder Elisa-Kupplung), insbesondere mit einer Magnetschicht, die das Ankoppeln von immunen Magnetköpfen erlaubt.

**18.** Vorrichtung nach einem der vorausgegangenen Ansprüche, wobei die Düsenplatte versehen ist mit einer Sol/Gel-Ultrafiltrationsbeschichtung oder einer gasdurchlässigen Schicht, umfassend Palladium.

**19.** Verfahren zum Herstellen kleiner Schattenmuster auf einem Substrat unter Anwendung der Vorrichtung gemäß einem der Ansprüche 1 bis 18.

**20.** Verfahren zum Filtern von Bier oder Milch durch Anwendung der Vorrichtung gemäß einem der Ansprüche 1 bis 18.

**21.** Verfahren zum Schäumen oder Emulgieren eines ersten Fluids durch Verwenden einer Vorrichtung gemäß einem der Ansprüche 1 bis 18, wobei der Hohlraum ein erstes Fluid aufnimmt, das ausgestoßen wird durch den ersten Bereich von Öffnungen an der Hauptfläche, und wobei die Düsenplatte in Kontakt mit einem zweiten Fluid an der Hauptfläche gebracht wird.

**22.** Verfahren nach Anspruch 21, wobei die Düsenplatte tangential und/oder rechtwinklig zu dem zweiten Fluid mit der Düsenplatte hin bewegt wird.

**23.** Verfahren nach Anspruch 22, wobei eine doppelte Emulsion durch Anwendung der Düsenplatte gebildet wird.

**Revendications**

**1.** Dispositif (1) pour la filtration et/ou l'émulsion d'un fluide, comprenant une plaque à buses (10, 15, 40, 50) et une structure de soutien de la plaque à buses (12, 17), ladite structure de soutien de la plaque à buses soutenant ladite plaque à buses (10, 15) sur une surface principale, dans lequel ladite plaque à buses possède une épaisseur de moins de 2 microns et comprend une pluralité d'orifices de buses (11, 16, 51, 61) s'étendant sur ladite épaisseur sur toute ladite plaque à buses, dans lequel la structure de soutien comprend une cavité (13) et ladite pluralité d'orifices de buses sont collectivement en communication fluidique avec ladite cavité, et dans lequel un orifice de buse au sein de ladite pluralité d'orifices de buses possède une longueur représentant moins de six fois son diamètre, et en particulier est plus court que son diamètre, **caractérisé en ce que** lesdits orifices de buses de ladite pluralité d'orifices de buses sont positionnés dans une première zone (61, 101) de ladite plaque de buses, **en ce que** ladite plaque de buses comprend une seconde zone (100) contiguë à ladite première zone qui est au moins substantiellement exempte de tout orifice de buse, **en ce que** ladite cavité (13) s'étend sous ladite première zone et ladite seconde zone de ladite plaque à buses, et **en ce que** ladite seconde zone possède une largeur qui est au moins plusieurs fois plus large que ladite épaisseur de ladite plaque à buses.

**2.** Le dispositif selon la revendication 1, dans lequel au moins un orifice (11, 16) de ladite plaque à buses (10, 15) possède un diamètre se situant entre 0.4 et 10 microns.

**3.** Le dispositif selon les revendications 1 ou 2, dans lequel un espacement mutuel (62) entre un orifice et un orifice adjacent possède un diamètre entre trois et trente fois supérieur au diamètre desdits orifices.

**4.** Le dispositif selon l'une quelconque des revendications précédentes, dans lequel ladite plaque à buses comprend plusieurs orifices substantiellement identiques qui sont séparés les uns des autres d'une distance mutuelle (62) qui se situe entre 3 et 30 fois, en particulier entre 3 et 10 fois, leur diamètre.

**5.** Le dispositif selon l'une quelconque des revendications précédentes, dans lequel ladite plaque à buses possède une surface nue et dans lequel ledit orifice dépasse légèrement de ladite surface nue de ladite plaque à buses.

**6.** Le dispositif selon l'une quelconque des revendications précédentes, dans lequel, sur ladite surface principale, ladite cavité possède une section transversale d'une largeur de moins de 250 microns, en particulier moins de 100 microns.

**7.** Le dispositif selon la revendication 6, dans lequel ladite section transversale possède une longueur de plus de 300 microns.

**8.** Le dispositif selon l'une quelconque des revendications précédentes, dans lequel ladite structure de soutien comprend un substrat de silicone, en particulier formé d'une pastille de silicium de <110>.

**9.** Le dispositif selon la revendication 8, **caractérisé en ce que** ladite plaque à buses est constituée d'une couche distincte appliquée sur ledit substrat, en particulier une couche de nitrure de silicium ou une couche de carbure de silicium.

**10.** Le dispositif selon l'une quelconque des revendications précédentes, dans lequel un substrat de verre est collé à ladite plaque à buses et dans lequel ledit substrat de verre comprend au moins un canal d'écoulement qui est en communication ouverte avec ladite cavité dans ladite structure de soutien soutenant ladite plaque à buses.

**11.** Le dispositif selon l'une quelconque des revendications précédentes, dans lequel ladite structure de soutien est suspendue dans un cadre de soutien en forme d'anneau qui est adapté aux dimensions respectives d'un porte-filtre disponible sur le marché.

**12.** Le dispositif selon la revendication 11, dans lequel ladite plaque à buses est enfoncée à une profondeur se situant entre 10 et 500 microns dans le cadre de soutien en forme d'anneau.

**13.** Le dispositif selon l'une quelconque des revendications précédentes, dans lequel une plaque de recouvrement transparente optique est placée au-dessus de la plaque à buses et dans lequel un canal d'écoulement d'une profondeur se situant entre 50 et 500 microns est présent entre la plaque à buses et la plaque de recouvrement.

**14.** Le dispositif selon l'une quelconque des revendications précédentes, dans lequel la plaque à buses est équipée d'une couche métallique permettant l'électrolyse ou des applications de chauffage électrique à non-transparence optique, sans trempe.

**15.** Le dispositif selon l'une quelconque des revendications précédentes, dans lequel la plaque à buses et la structure de soutien de la plaque à buses sont recouvertes d'un revêtement résistant à la corrosion, en particulier un revêtement de nitrure de silicium.

**16.** Le dispositif selon l'une quelconque des revendications précédentes, dans lequel la plaque à buses est équipée d'un dispositif d'actionnement piézoélectrique.

**17.** Le dispositif selon l'une quelconque des revendications précédentes, dans lequel la plaque à buses est équipée d'un agent immuno-liant (ou couplage Elisa), en particulier une couche magnétique permettant le couplage de billes immuno-magnétiques.

**18.** Le dispositif selon l'une quelconque des revendications précédentes, dans lequel la plaque à buses est équipée d'un revêtement d'ultrafiltration sol/gel ou d'une couche perméable au gaz comprenant du palladium.

**19.** Méthode consistant à pratiquer de petits motifs virtuels sur un substrat en utilisant le dispositif tel que revendiqué dans les revendications 1-18.

**20.** Une méthode de filtration de la bière ou du lait en utilisant le dispositif tel que revendiqué dans l'une quelconque des revendications 1-18.

**21.** Une méthode de moussage ou d'émulsion d'un premier fluide en utilisant le dispositif tel que revendiqué dans l'une quelconque des revendications 1-18, dans laquelle ladite cavité accueille un premier fluide qui est éjecté à travers *ladite première zone d'orifices* sur ladite surface principale et dans laquelle ladite plaque à buses est mise en contact avec un second fluide sur ladite surface principale.

**22.** La méthode selon la revendication 21, dans laquelle ladite plaque à buses est déplacée de manière tangentielle et/ou orthogonale par rapport audit second fluide en contact avec la plaque à buses.

**23.** La méthode selon la revendication 22, dans laquelle une double émulsion est formée à l'aide de ladite plaque à buses.

Fig. 1

Fig. 2

Fig. 3

Fig. 4

Fig. 5A

Fig. 5B

Fig. 6

Fig. 7A

Fig. 7B

51

50

Fig. 8A

52

55

Fig. 8B

Fig. 8C

56

Fig. 9

Fig. 10A

**Fig. 10B**

**Fig. 11**

**Fig. 12**

**Fig. 13**

Fig. 14

Fig. 15

Fig. 16

Fig. 17

Fig. 18

EP 2 177 272 B1

**Fig. 19**

**Fig. 20**

27

221

220

**Fig. 21**

223

221

222

220

**Fig. 22**

Fig. 23

Fig. 24

**Fig. 25**

**Fig. 26**

**Fig. 27A**

Fig. 27B

Fig. 28

## REFERENCES CITED IN THE DESCRIPTION

**Patent documents cited in the description**

• US 6086195 A

**Non-patent literature cited in the description**

• **DAGAN et al.** *Chem.Eng.Svi.,* 1983, vol. 38, 583-596